# EUROPEAN PATENT APPLICATION

(11) **EP 1 600 443 A1**
(43) Date of publication of application: **30.11.2005**
(21) Application number: 04425379.7
(22) Date of filing: 26.05.2004
(51) Int. Cl.: C07D 223/26, C07D 223/28

(54) **Process for the preparation of oxcarbazepine**

(71) Applicant: Milanese, Alberto, 20052 Monza (IT)
(72) Inventor: Milanese, Alberto, 20052 Monza (IT)
(74) Representative: Bianchetti, Giuseppe

(57) **Abstract**

The reaction between 10-methoxy-iminostilbene (IV) and of(trichloromethyl)carbonate affords 10-methoxy-N-chlorocarbonyliminostilbene (XVI) in high yields, then ammonolysis and subsequent hydrolysis of the enol ether provides particularly pure oxcarbazepine.

## Description

The present invention relates to a process for the preparation of iminostilbene derivatives.

More particularly, the invention relates to a process for the preparation N-chlorocarbonyl-10-methoxy-iminostilbene of formula XVI by reaction of the corresponding 10-methoxy-iminostilbene with triphosgene, in the presence of bases B (which will be defined in the following), according to scheme:

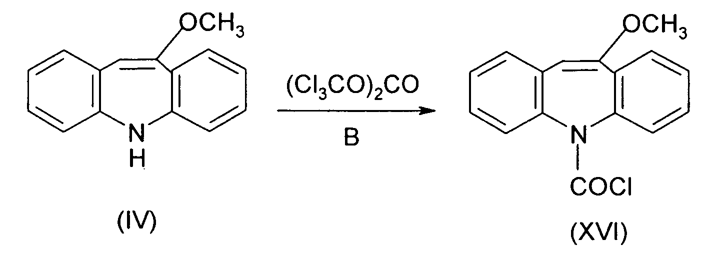

A further object of the invention is a process for the preparation of 10-oxo-10,11-dihydro-5H-dibenz(b,f)azepine-5-carboxamide VI by reaction of the chlorocarbonyl derivative XVI with ammonia and acid hydrolysis of the resulting N-aminocarbonyl-10-methoxy-iminostilbene V, according to the scheme:

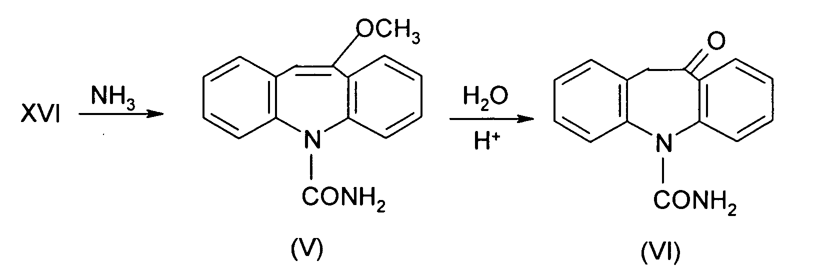

Compound VI is an active ingredient well known under the name oxcarbazepine (see "The Merck Index", 13^{th} edition, 6998).

A number of alternative processes for the preparation of oxcarbazepine are already known. One of them (DE 2.246.842, CH 633.271) involves the epoxidation of carbazepine VII and the rearrangement of the epoxide VIII to oxcarbazepine VI

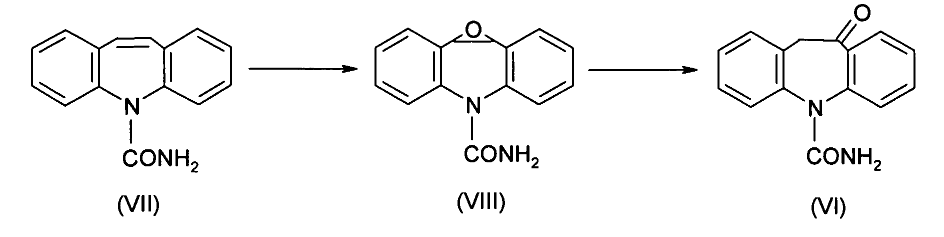

The main drawback of this process is the use of carbamazepine as reagent, as it a castly finished product. Furthermore, this substrate is very unstable so that the epoxidation reaction takes place in low yields when using common epoxidants such as peracetic acid, or it requires remarkable excesses of expensive reagents, such as 3-chloroperbenzoic acid. Moreover, dangerous by-products form during epoxidation. The rearrangement reaction epoxide → ketone takes place with large amounts of costly catalysts and is difficult to control, leading to formation of a number of by-products, thus, the crude requires thorough purifications and yields are poor.

According to another process (EP-A-0 028 028) N-cyano-iminostilbene IX is subjected to nitration; the resulting 10-nitro-N-cyano-iminostilbene X is reduced to 10-amino-N-cyano-iminostilbene which, by hydrolysis of the enamine to ketone XI and of the group CN to CONH₂, yields oxcarbazepine IV, according to the scheme D:

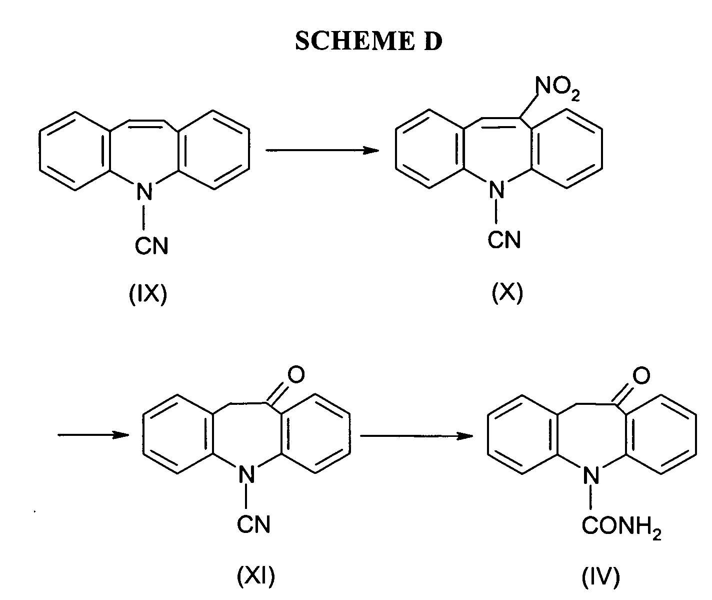

The limits of this process basically consist in the starting product IX which can be obtained, according to EP-A-0029409, either from expensive carbamazepine or from 5H-dibenz-(b,f)azepine (iminostilbene, I) by reaction with cyanogen chloride, a difficult to handle-toxic-gas:

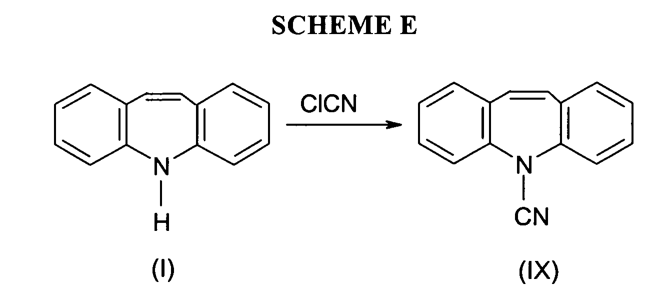

Moreover, the nitration provides acceptable yields only when carried out with nitrating agents such as N₂O₃ or N₂O₄, which are difficult to use. Finally, hydrolysis of the cyano group preferably requires the use of BF₃ complexes, which are expensive and difficult to handle as they are very aggressive. The total process yield is low.

According to CH 642.950, 10-chloro-5H-dibenz(b,f)azepine carboxamide XII is hydrolysed with concentrated sulfuric acid, to give directly oxcarbazepine.

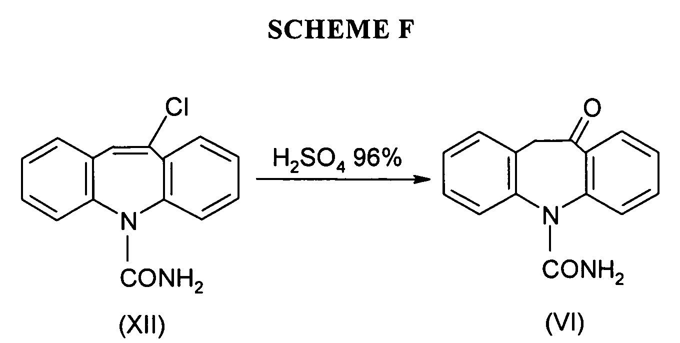

However, the hydrolysis is extremely slow and remains incomplete at the cited temperature, whereas an even slight increase in temperature induces degradation.

Moreover, the preparation of XII requires the treatment of 10-chloro-5H-dibenz(b,f)azepine (XIII) with toxic phosgene gas or the use of carbamazepine VII as the starting reagent, according to schemes G and G':

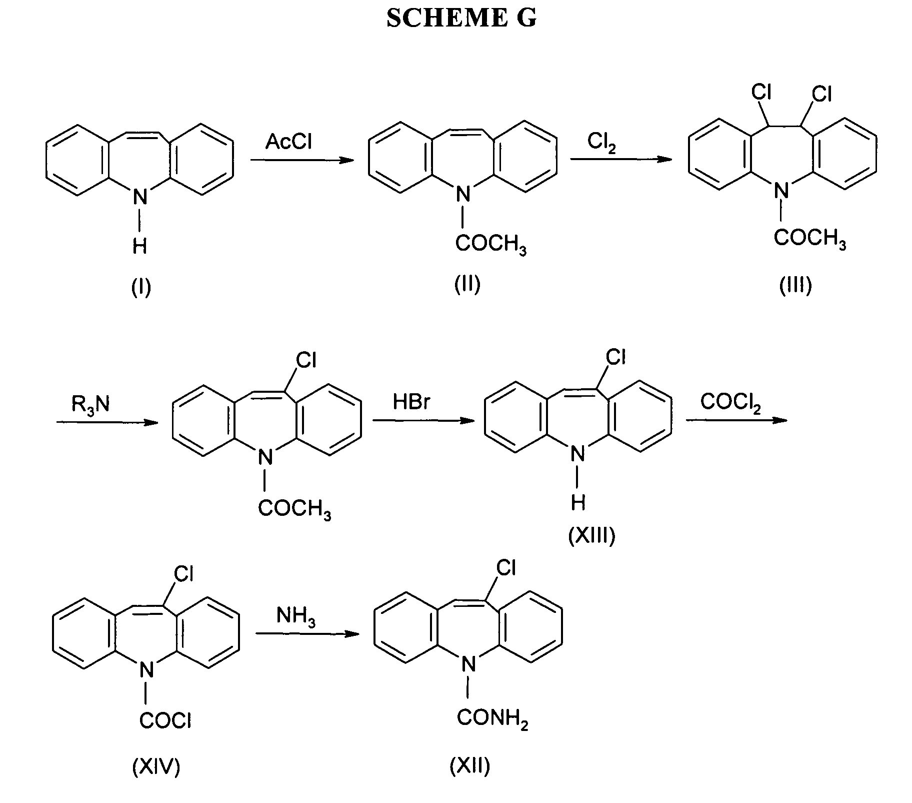

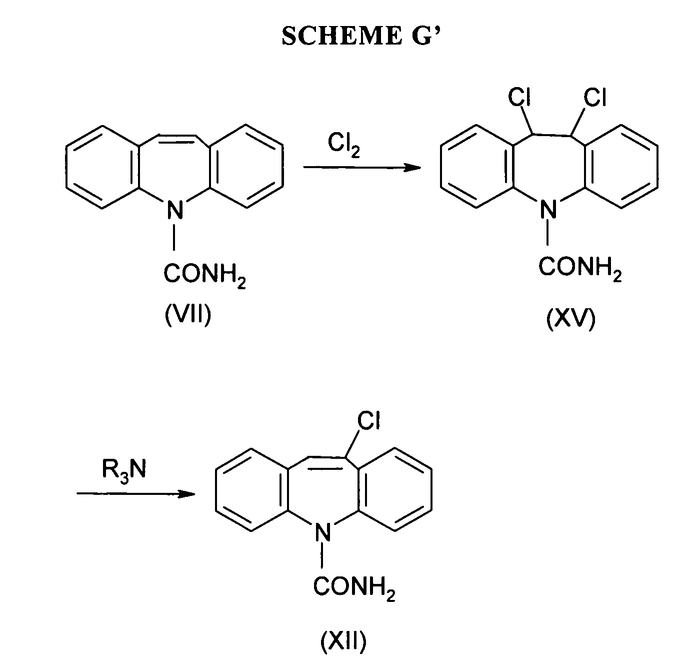

The process according to Scheme G involves a large number of steps, while that of scheme G' makes use of starting material VII which is very expensive.

According to German Patent 2.011.087 (Scheme H), treatment of the 10-methoxy-iminostilbene IV with phosgene should afford the corresponding N-chlorocarbonyl derivative (XVI), which should give oxcarbazepine by ammonolysis and hydrolysis.

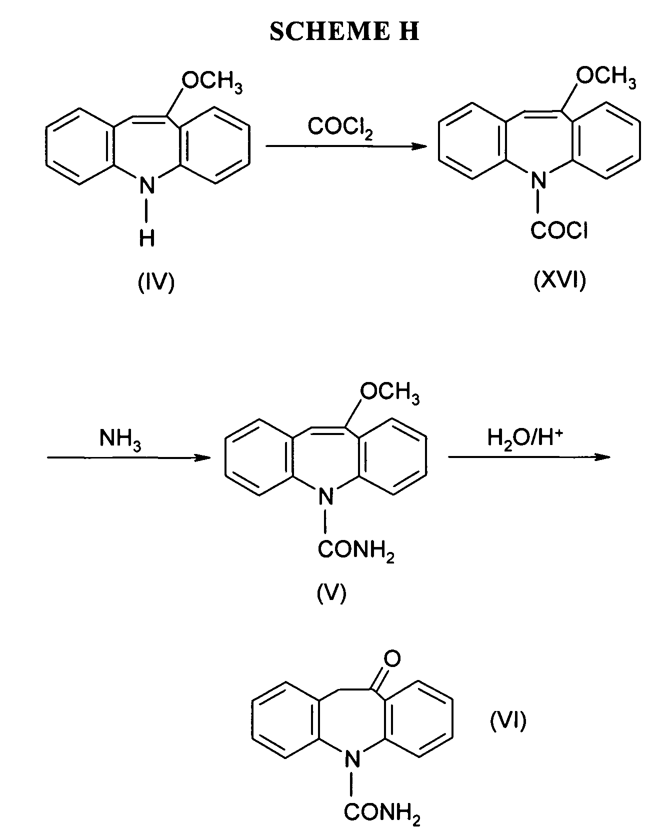

Conversely, the expected phosgenation product does dot form, the reaction leading to 10-ketoiminostilbene, which, being inert to phosgenation, increases the dangerousness of the process.

More recently EP 847.390 has disclosed a process in which 10-methoxy-iminostilbene IV is reacted with alkali or alkaline-earth cyanates in the presence of acids, to give 10-methoxy-N-aminocarbonyl-iminostilbene (V) which is subjected to acid hydrolysis

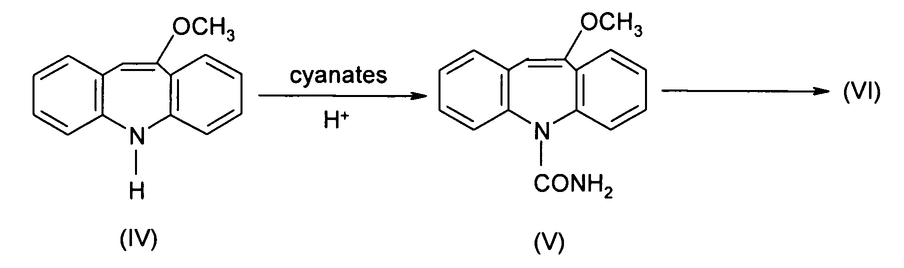

Although the latter process proves superior to those mentioned above, this often yields the product together with unidentified impurities, thus requiring complex purification procedures.

It has now been found that, contrary to the results obtained using phosgene according to the process disclosed in DE 2.011.087 (see above, scheme H), the reaction between 10-methoxy-iminostilbene (IV) and triphosgene (bis(trichloromethyl) carbonate) in the presence of bases B affords the desired N-chlorocarbonyl derivative (XVI) in good yields, and that this can be easily transformed into 10-methoxy-N-aminocarbonyl-iminostilbene (V), which by acid hydrolysis gives oxcarbazepine (scheme K):

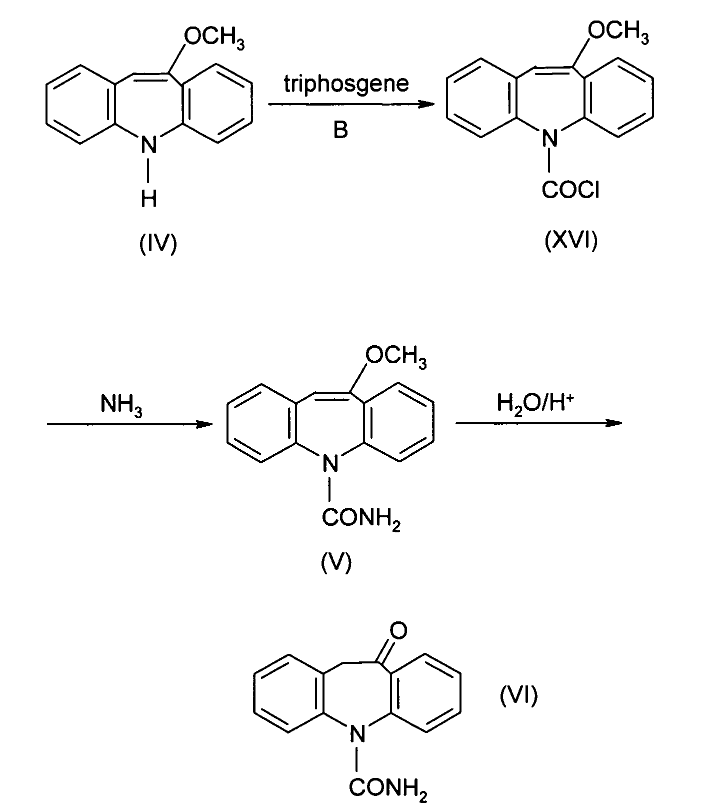

The chlorocarbonylation reaction is carried out in the presence of bases B, preferably of tertiary bases such as triethylamine, pyridine, diisopropylethylamine, 1,8-diazabicyclo [2.2.2]octane, 1,8-diazabicyclo [5.4.0]undec-7-ene, 1,5-diazabicyclo [4.3.0]non-5-ene and the like. Fairly good results are also obtained with alkali or alkaline-earth carbonates; however, the above organic bases are preferred, in particular triethylamine.

The starting 10-methoxy-iminostilbene can be easily obtained starting from iminostilbene according to BE 597.793 and US Re 27.622.

The reaction is carried out in aprotic solvents such as toluene, xylene, benzene, or in ethers or chlorinated solvents, at temperatures ranging from about -10°C to the reflux temperature of the solvent. The molar ratio of 10-methoxy-iminostilbene to triphosgene can range from 3:1 to 1:1; the base is used in at least equimolar amounts to the formed hydrochloric acid. Triphosgene can be added to the 10-methoxy-iminostilbene solution, already containing the organic base, either gradually or in a single portion, as such or dissolved in a solvent; or the triphosgene solution can be added with a 10-methoxyiminostilbene solution containing the required base. 10-Methoxy-N-chlorocarbonyl-iminostilbene can be isolated and optionally purified according to known methods, or it can be directly subjected to ammonolysis by treatment of the reaction solution with anhydrous ammonia dissolved in suitable solvents, or with aqueous ammonia.

In the last step, enol ether (V) is hydrolysed to carbonyl group, thereby obtaining oxcarbazepine (VI), by heating with diluted mineral acids (such as 10% H₂SO₄, as disclosed in example 1 of EP 847.390) or with organic acids, for example formic, acetic, trichloroacetic and the like, in the presence of water. The resulting oxcarbazepine is purified by simple crystallization according to the above mentioned patents, suitably with dimethylformamide and alcohols or dimethylformamide and ketones.

As already mentioned, the process of the invention affords the intermediate 10-methoxy-N-chlorocarbonyl-iminostilbene (XVI) in pure form, thus providing particularly high yields, not lower than 85% on theoretical, thanks to the reagent which is by far less aggressive than phosgene (which, as already mentioned, does not afford the desired product) and easier to handle; furthermore, said intermediate is pure enough to give in the successive reactions a particularly pure final product.

The following examples further illustrate the process of the invention.

### Example 1

a) A solution of 66.9 g (0.3 mols) of 10-methoxy-iminostilbene and 34.92 g (0.34 mols) of triethylamine in 800 ml of toluene is gradually added, during 6 hours and at a temperature of 10-15°C, with a solution of 32.67 g (0.11 mols) of triphosgene in 300 ml of toluene. After completion of the reaction (disappearance of methoxy-iminostilbene) 200 ml of 30% aqueous ammonia are added gradually, vigorously stirring at room temperature for some hours. After that, the phases are separated, the toluene phase is washed with water and evaporated to dryness under reduced pressure. Yield: 69.0 g (85% on theoretical) of 10-methoxy-N-aminocarbonyl-iminostilbene (V) of purity higher than 95%.
b) The resulting product is hydrolysed by refluxing with 100 ml of 10% H₂SO₄ for an hour. After cooling to room temperature, filtration and washing with water, the reaction mixture is recrystallized from dimethylformamide, and the precipitate is washed with acetone and dried under vacuum. 57.0 g of oxcarbazepine (yield 75.3% on theoretical) are obtained, the structure being confirmed by IR, NMR and mass spectroscopies.

### Example 2

Following the procedure of Example 1, but using triphosgene in equimolar amount to 10-methoxyiminostilbene, and proportionally increasing Triethylamine, 58.5 g of oxcarbazepine are obtained (77.3% on theoretical).

### Example 3

Following the procedure of Example 1, but using an equimolar amount of pyridine instead of triethylamine, the yield is 55.0 g (72.7% on theoretical).

### Example 4

Following the procedure of Example 1, but using 30% aqueous formic acid instead of the same volume of 10% aqueous sulfuric acid, the yield is 58.0 (76.6% on theoretical).

### Example 5

Following the procedure of Example 4, but using dichloromethane as the solvent instead of toluene, at the reflux temperature, the yield is 52.0 (68.7% on theoretical).

### Example 6

Following the procedure of Example 1, but dropping the solution of 10-methoxyiminostilbene and triethylamine into the solution of triphosgene in toluene, in 2 hours, the yield is 54.7 (72.2% on theoretical).

### Example 7

The procedure of Example 6 is followed, but using 1,4-diazabicyclo-[2.2.2]octane is used instead of an equivalent amount of triethylamine. The acid hydrolysis is carried out as in Example 4. The yield is 51.0 g (67.3% on theoretical).

## Claims

1. A process for the preparation of 10-oxo-10,11-dihydro-5H-dibenz(b,f)azepine-5-carboxamide VI (oxcarbazepine VI) comprising the following steps:
a) reaction of 10-methoxy-iminostilbene IV with triphosgene in the presence of bases, to give 10-methoxy-N-chlorocarbonyl-iminostilbene XVI;
b) ammonolysis of 10-methoxy-N-chlorocarbonyl-iminostilbene XVI, to give 10-methoxy-N-aminocarbonyl-iminostilbene V;
c) acid hydrolysis of the resulting 10-methoxy-N-aminocarbonyl-iminostilbene V, to give oxcarbazepine VI,
according to the following scheme:

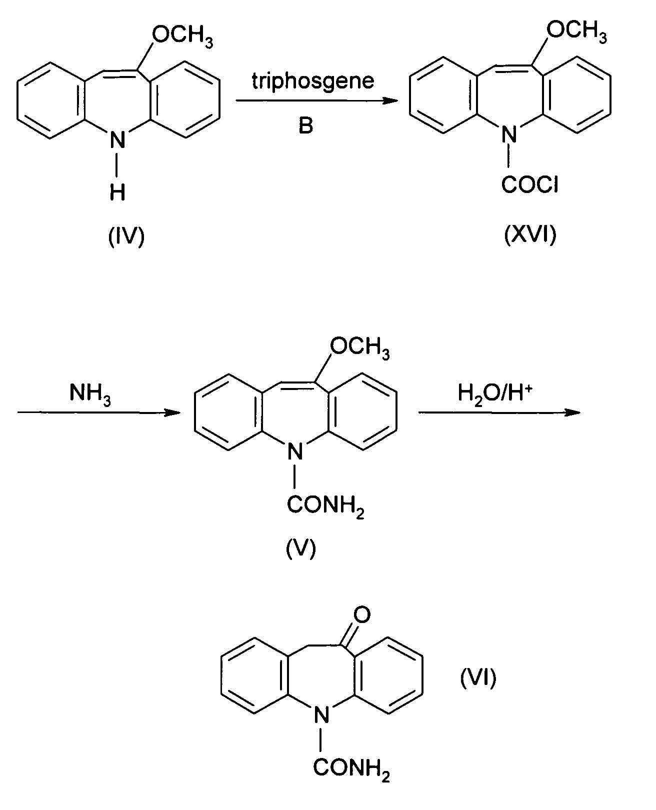

2. A process as claimed in claim 1, wherein reaction a) is carried out in apolar solvents selected from toluene, xylene, benzene, ethers and chlorinated solvents, at temperatures ranging from -10°C to the reflux temperature of the solvent.

3. A process as claimed in claims 1 and 2, wherein in reaction a) tertiary bases selected from triethylamine, pyridine, diisopropylethylamine, 1,4-diazabicyclo[2.2.2]octane, 1,8-diazabicyclo[5.4.0]undec-7-ene, 1,5-diazabicyclo [4.3.0]non-5-ene and the like and alkali or alkaline-earth carbonates are used.

4. A process according to the above claims, wherein triethylamine is used.

5. A process according to the above claims, wherein the molar ratio of 10-methoxy-iminostilbene to triphosgene ranges from 3:1 to 1:1.

6. A process according to the above claims, wherein the base is used in at least equimolar amounts to the formed hydrochloric acid.

7. A process according to the above claims, wherein triphosgene, optionally dissolved in apolar solvents, is gradually added to the solution of 10-methoxy-iminostilbene already containing the calculated amount of base, or said solution is added to the triphosgene solution.

8. A process as claimed in claim 1, wherein the solution of 10-methoxy-N-chlorocarbonyl-iminostilbene is directly added with anhydrous ammonia or ammonia dissolved in a solvent.

9. A process as claimed in claim 8, wherein aqueous ammonia is used.

10. A process as claimed in claim 1, wherein hydrolysis of 10-methoxy-N-aminocarbonyl-iminostilbene is carried out with diluted mineral acids.

11. A process as claimed in claim 1, wherein hydrolysis of 10-methoxy-N-aminocarbonyl-iminostilbene is carried out with organic acids selected from formic, acetic, trichloroacetic acids, in the presence of water.

12. Oxcarbazepine obtained according to the process of claims 1-11, after crystallization from dimethylformamide and alcohols or ketones, selected from methanol, ethanol, isopropanol, n-propanol, acetone, 2-butanone.
